# EUROPEAN PATENT SPECIFICATION

(11) **EP 3 643 181 B1**
(45) Date of publication and mention of the grant of the patent: **26.05.2021**
(21) Application number: 19182605.6
(22) Date of filing: 26.06.2019
(51) Int. Cl.: A23K 10/24, A23J 3/34, A61K 38/06, A61K 38/05, A61K 38/01, A23J 3/12, A23J 1/06, A23L 3/18, A23L 33/17, A61K 35/16, A61K 38/38, A61P 3/02

(54) **METHOD FOR THE MANUFACTURING OF A FOOD SUPPLEMENT**
VERFAHREN ZUR HERSTELLUNG EINES NAHRUNGSERGANZUNGSMITTELS
PROCÉDÉ DE FABRICATION D'UN COMPLÉMENT ALIMENTAIRE

(30) Priority: 26.10.2018 BE 201805741
(43) Date of publication of application: 29.04.2020
(73) Proprietor: Dagon Products BVBA, 9420 Erpe-Mere (BE)
(72) Inventor: D'Herde, Wim, 9420 Erpe-Mere (BE)
(74) Representative: Brantsandpatents bvba

(56) References cited:
- EP-A2- 0 274 939
- WO-A1-99/07236
- WO-A1-2006/080624
- GB-A- 2 000 956
- US-A- 2 996 383
- US-A- 3 706 571
- US-A- 4 067 119
- US-A- 5 356 637

## Description

### TECHNICAL DOMAIN

The method relates to the fabrication of a food supplement.

More in particular, the invention relates to the technical subdomain of an adaptogenic food supplement.

### STATE OF THE ART

The use of human and/or animal blood fractions as a supplementary food source for the fight against undernourishment, or in particular the protein undernourishment, in developing countries is a well-known practice since a long time. Blood is rich in high-quality proteins and iron, and is thus very appropriate for this purpose.

Blood fractions or derivatives thereof can however also mean a surplus value in less extreme cases of undernourishment, or with other kinds of physical stress. For example, certain stressful periods can require a particular support, for example when recovering from a sport injury. In such situations, a food supplement is an appropriate dosage form. The food supplement is ideally in the form of a powder, capsule or tablet, which can easily be taken by a subject.

An appropriate method for drying a blood fraction, for forming a food supplement, is spray-drying. For example, US 9 867 782 discloses a method for drying blood plasm. US '782 has however mainly as a goal to obtain a dry blood fraction which can be kept for a long time, for re-hydrating this blood fraction after storage. This is namely useful for blood transfusions. The biological activity when taking such dry blood fraction by a subject is hereby however not guaranteed.

US 6 004 576 discloses a similar method, in which animal blood plasm is dried for producing an animal feed supplement. However, the field of application of US '576 has mainly as a goal to cause a weight increase in animals. The focus is here thus mainly on the energetic value of the dry blood fraction, and not on its qualitative characteristics or biological activity

US 2 996 383 A discloses a process in which dried animal blood is subjected to moist heat in a pressure cooker at a pressure of from 86 kPa (12.5 psig) to 155 kPa (22.5 psig) for 30 minutes to 2 hours. The heat treatment partially hydrolyses the protein, effecting an improvement in the digestibility and biological value.

Thus, there is a need for a method for fabricating a food supplement from a blood fraction, in which the food supplement has a high purity and shows a high biological activity.

The present invention aims to find at least a solution for some of the above-mentioned problems or disadvantages.

### SUMMARY OF THE INVENTION

Thereto, the present invention provides a method for the fabrication of a food supplement of claim 1. The method comprises the provision of a blood fraction and the drying of the blood fraction, in which the blood fraction is subjected to a high-intensity heat treatment after drying. Said high-intensity heat treatment is carried out at a temperature between 80.0 and 110.0°C and a pressure between 200 and 500 kPa, for 0.1 to 10.0 seconds.

Preferred embodiments of the method are described in the dependant claims 2 to 8.

The method causes an efficient conversion of a liquid blood fraction into a dry, powder fraction under the influence of spray-drying, which powder fraction is appropriate as a base for a food supplement with adaptogenic characteristics. The specific heat treatment breaks down the protein components in the dry blood fraction into amino acids, di-, tri- and polypeptides, as a result of which they can be better absorbed, and be better metabolised by the body of the subject.

A specific embodiment relates to the method of claim 4. According to this preferred embodiment, the high-intensity heat treatment is followed by a fractionation at a maximal particle size of 750 µm. Fractioning to this particle size allows to easily pelletize the food supplement.

Another preferred embodiment relates to the method in which the serum albumin is obtained by means of the fractionation of cattle and/or pig blood.

The food supplement comprises a protein component that is derived from a blood fraction of one or more free amino acids, in which the protein component further comprises di- and/or tripeptides that are derived from a blood fraction.

Di- and tripeptides can be better absorbed and better metabolised by the body of a subject than longer polypeptides and/or more complex proteins. A concentration of at least 80 m% of di- and/or tripeptides consequently implies a high biological activity of the food supplement. The food supplement hereby shows excellent adaptogenic characteristics with respect to a subject taking the food supplement.

There is also described a food supplement, in which the minerals comprise iron (Fe), preferably heme iron (Fe), more preferably heme iron in the form of hemin. Iron deficiency is usually also at the basis of reduced mental and/or physical performances, therefore supplementing this deficiency further supports the adaptogenic function of the food supplement.

The food supplement can be used for cosmetic and sport purposes. Cosmetic applications comprise amongst other things the improvement of hair and nail quality, and the deceleration of aging. Sport applications are amongst other things directed to the improvement of sport performances.

### DETAILED DESCRIPTION

The present invention relates to a method for the fabrication of a food supplement comprising the provision of a blood fraction of a subject, for example a mammal, and the drying of the blood fraction. After drying, the dried blood fraction is subjected to a high-intensity heat treatment at a temperature between 80.0 and 110.0°C and a pressure between 200 and 500 kPa. The duration of the high-intensity heat treatment is significantly reduced, and is namely comprised between 0.1 and 10.0 seconds.

Unless otherwise specified, all terms used in the description of the invention, including technical and scientific terms, shall have the meaning as they are generally understood by the worker in the technical field of the invention. For a better understanding of the description of the invention, the following terms are explained specifically.

"A", "an" and "the" refer in the document to both the singular and the plural form unless clearly understood differently in the context. "A segment" means for example one or more than one segment.

When "approximately" or "about" are used in the document together with a measurable quantity, a parameter, a period or moment, etc., variations of +/-20% or less, preferably +/-10% or less, more preferably +/-5% or less, still more preferably +/-1% or less, and even still more preferably +/-0.1% or less than and of the cited value are meant, as far as such variations apply to the invention that is described. It will however be clearly understood that the value of the quantity at which the term "approximately" or "about" is used, is itself specified.

The terms "include", "including", "consist", "consisting", "provide with", "contain", "containing", "comprise", "comprising" are synonyms and are inclusive of open terms that indicate the presence of what follows, and that do not exclude or prevent the presence of other components, characteristics, elements, members, steps, known from or described in the state of the art.

The citation of numeric intervals by means of end points includes all integers, fractions and/or real numbers between the end points, including these end points.

The term "food supplement" means a food that is meant as a supplement to the normal nutrition. It is a concentrated source of possibly one or more vitamins, minerals or other nutrients with a nutritional and/or physiological effect. Food supplements are situated in the transitional field between food and over-the-counter drugs (OTC). Firstly, because its dosage form is often identical to that of drugs, namely in the form of capsules, tablets, powders, sachets and similar. Secondly, food supplements are also often used for therapeutic purposes (prevention and treatment of diseases).

The food supplement is of a "natural" and/or "biological" origin. Both terms should be interpreted in the context of the medical and/or pharmaceutical sector as obtained from vegetable, animal or other natural sources, provided that they have been subjected to one or more extraction and/or separation steps.

By extension, the method according to the present invention is also appropriate for fabricating a feed additive.

A "blood fraction" means in the context of the present invention a single and/or compound product, which product can be obtained directly from animal blood. For example, a blood fraction can possibly comprise blood plasm, blood serum, electrolytes, colloidal components, nutrients or other components that can be found in animal blood.

In the framework of the present invention, the term "heat treatment", and in particular "high-intensity heat treatment", is used to indicate a short, however intense heating. Such heating possibly takes place under increased pressure.

The high-intensity heat treatment of the method of the present invention is carried out at a temperature between 80.0 and 110.0 °C and a pressure between 200 and 500 kPa. The duration of the high-intensity heat treatment is comprised between 0.1 and 10.0 seconds. The method causes an efficient conversion of a liquid blood fraction into a dry, powder fraction under the influence of spray-drying, which powder fraction is appropriate as a base for a food supplement with adaptogenic characteristics.

"Adaptogenic characteristics" means that, when the subject takes the supplement, this subject is better resistant to changing environmental factors and/or stressful stimuli. Adaptogenic characteristics possibly comprise stimulating the immune system, increasing the stress resistance, stimulating the physical activity, countering cell aging, fighting fatigue, improving hair and nail quality, supporting vital organs, such as the liver, and/or improving the capacity to concentrate.

The use of a blood fraction for the fabrication of a food supplement has the advantage that blood proteins are abundant, cheap and easily available. By nature, they have an optimal amino acid profile. Moreover, blood is a main source of iron. Since iron deficiency is the most common nutritional deficiency in the world, this is a large advantage of the resulting food supplement.

The fabrication of a food supplement in a dry form simplifies the taking of the supplement by a human subject. The biological activity of such a dry blood fraction is drastically increased under the influence of a heat treatment. Such heat treatment breaks down the protein components in the dry blood fraction into amino acids, di-, tri- and polypeptides, as a result of which they can be better absorbed, and be better metabolised by the body of the subject. The breakdown is particularly characterized by an N-terminal degradation of the protein component. N-terminal degradation takes place naturally, but it is however strongly favoured and accelerated by said high-intensity heat treatment. As a result of N-terminal degradation, a large quantity of dipeptides is released, showing a high biological activity.

Appropriate methods for isolating, analysing and identifying dipeptides comprise High Performance Liquid Chromatography (HPLC), in particular Reserve-Phase High Performance Liquid Chromatography (RP-HPLC), Nuclear Magnetic Resonance (NMR) and N-terminal Sequencing.

A heat treatment that lasts too long and/or at too high temperature causes a further breakdown of the proteins that are present in the blood fraction, as a result of which only free amino acids remain in the dry fraction. This is disadvantageous for the activity of the food supplement. An optimal conversion is obtained at a high-intensity heat treatment that is carried out at a temperature between 80.0 and 110.0 °C and a pressure between 200 and 500 kPa. At a heat duration between 0.1 and 10.0 seconds, an optimal conversion is obtained, in which mainly di- and tripeptides are present in the dry fraction. It is in particular these di- and tripeptides that offer excellent adaptogenic characteristics to the resulting food supplement.

Preferably, the high-intensity heat treatment is carried out at a temperature between 85.0 and 95.0 °C and a pressure between 250 and 350 kPa. The duration of the high-intensity heat treatment is hereby comprised between 0.2 and 1.0 seconds. A short and intense heat treatment results in a larger content of di- en tripeptides in the dry fraction, with a higher biological activity of the supplement as a result. More preferably, the temperature of the high-intensity heat treatment is comprised between 88.0 and 95.0°C. The pressure is more preferably comprised between 280 and 320 kPa. The duration of the high-intensity heat treatment can further be shortened within said temperature and pressure range. More preferably, the duration of the high-intensity heat treatment is less than 0.8 seconds. Most preferably, the method comprises a high-intensity treatment carried out at a temperature between 90.0 and 95.0°C and a pressure of about 300 kPa, for at maximum 0.6 seconds.

According to a further or other embodiment, the drying of the blood fraction takes place by means of spray- and/or freeze-drying.

The term "spray-drying" refers to the industrial method of forming a powder from a solution. The concentrated solution is led through a sprayer, and subsequently atomized in a heated tower. By atomizing and drying in this way, one obtains a powder with a relatively small grain size. Spray-drying is used frequently at the fabrication of amongst other things milk powder, instant coffee and washing powders.

The term "freeze-drying", or also called "lyophilisation", consists of a drying process at low temperature in which the product is frozen, the pressure is reduced and subsequently the ice is removed by means of sublimation. Freeze-drying results in a high-quality product because of the low temperature that is used at the processing. The original form of the product is maintained, and the quality of the dried product is particularly good.

According to a further or other embodiment, the dried blood fraction undergoes a dehydration, which precedes the high-intensity heat treatment. In the context of the present invention, "dehydration" refers to a long-term heating at low intensity, in which small amounts of water are removed from an almost dry substance. Said dehydration is carried out at a temperature between 60.0 and 80.0 °C and at atmospheric pressure, during a period of 5 and 10 hours. Hereby, the dried blood fraction is further released from remaining fluid without changing the chemical structure. Thereto, a low temperature, namely within said range, is necessary as higher temperatures give rise to an uncontrolled breakdown of the proteins that are present in the blood fraction. Preferably, the temperature of the dehydration is comprised between 65.0 and 75.0°C. The advantage of a carefully dehydrated composition is an increased stability and an extended shelf life. Most preferably, the temperature is about 70.0°C and the dehydration is carried out for about 8 hours. Hereby, a full dehydration is realized, without an increased risk of uncontrolled protein breakdown.

An embodiment of the method comprises a fractionation following the high-intensity heat treatment. A "fractioning" means a separation process in which a certain portion of a mixture is separated in several smaller portions or fractions. Such fractioning takes place because several components of a mixture have different chemical and/or physical characteristics. A fractionation can for example be based on a difference in specific gravity, boiling point or particle size.

Said fractionation causes a separation at a maximum particle size of 750 µm. At a particle size of maximum 750 µm, the dry fraction can easily be pelletized into user-friendly pellets and/or pills. Preferably, a particle size of maximum 650 µm is aimed at, for further improving the pelletization. More preferably, the fractionation provides for a separation at a maximum particle size of 550 µm, most preferably 500 µm.

According to an embodiment, the dried blood fraction undergoes a purification, that follows the high-intensity heat treatment and that comprises one or more physical purification steps.

A "purification" refers to one or more processes in which impurities are removed from a product, as a result of which an end product is obtained with possibly improved characteristics. The purification preferably takes place by means of one or more physical purification steps.

According to a further or other embodiment, the drying takes place by means of spray-drying and the spray-drying precedes the high-intensity heat treatment. The spray-drying takes place at a temperature that is comprised between 170.0 and 190.0°C, for 25 to 35 minutes, most preferably at 180.0°C, for 30 minutes.

For obtaining optimal results, a preferred embodiment of the present method comprises the following successive steps: (a) providing a blood fraction, (b) spray-drying this blood fraction at a temperature between 170.0 and 190.0°C for 25 to 35 minutes, most preferably at 180 °C for 30 minutes, (c) dehydrating at a temperature between 65.0 and 75.0 °C and for 7 to 9 hours, most preferably at 70.0 °C for 8 hours, (d) high-intensity heat treatment, at a temperature between 90.0 and 95.0 °C and a pressure between 290 and 310 kPa, most preferably at 300 kPa, and this for 0.2 to 0.8 seconds, most preferably maximum 0.6 seconds, (e) fractioning into a maximum particle size of 500 µm, and finally, (f) physical purification.

The described successive treatment steps guarantee an optimal maintenance of the quality of the blood fraction throughout the realization of the method, and hereby ensures an efficient conversion of the blood fraction into a food supplement, which food supplement is rich in di- and tripeptides. The resulting food supplement hereby comprises a concentration of di- and tripeptides which is at least 80 m%. Consequently, the resulting food supplement shows a particularly high biological activity and particularly good adaptogenic characteristics.

The chemical composition, amino acid composition, energetic value, chemical toxicity, microbiological quality, density, particle size and/or other quality parameters of the blood fraction, the resulting food supplement, and/or intermediate products generated during the method are possibly controlled during the method, according to procedures that are known by the skilled worker.

Said quality parameters are a measure for the quality of the provided blood fraction, and they also constitute a control of the quality of realization of the method. The protein content and the moisture content are hereby determined by means of ISO 1442, the ash content by means of ISO 936, the total lipid content by means of ISO 1443, and the total quantity of dietary fibres is determined by means of AOAC 991.43. The energetic value is determined corresponding to EG 1169/2011.

Chemical toxicity is controlled with respect to toxic metals, such as cadmium, mercury, lead, arsenic, selenium, copper, zinc, or combinations thereof. Toxicity control also comprises a pesticide analysis.

The blood fraction as provided in the present method comprises serum albumin according to an embodiment.

The term "serum albumin" refers to a specific blood fraction comprising albumin, a globular protein that is produced by the liver. Albumin is the most common blood protein in mammals and is essential for the maintenance of the oncotic pressure, i.e. the pressure that is necessary for a correct distribution of body fluids between blood vessels and body tissue.

According to the present method, said serum albumin is obtained by means of the fractionation of cattle and/or pork blood. Both cattle and/or pork blood are ideally appropriate as a start product in the present method, considering their resemblances with human blood. By doing so, a particularly high biological activity of the obtained food supplement is realized, and the risk of side effects is minimized. The serum albumin, provided as a starting material in the present invention, should be of an excellent quality. In such way, different factors are subjected to a solid control. Thus, the serum albumin is selected based on a good health of the animals, good environmental and climatologic circumstances at the animal production, origin of slaughterhouses with a large number of slaughterings, purification and processing in sterile laboratories, or combinations thereof.

In this context, a commonly used blood fractionation technique for obtaining pure serum albumin is centrifugation. By centrifuging blood, usually three fractions are obtained: a fraction containing blood plasm, a fraction containing white blood cells and/or blood platelets, and a fraction containing red blood cells. The fraction containing blood plasm can further be separated into serum, comprising blood proteins that do not contribute to coagulation, electrolytes, antibodies, antigens and hormones on the one hand, and fibrinogens that contribute to coagulation on the other hand. A qualitative separation of blood, with a high-purity serum, and in particular, a high-purity serum albumin as a result, correspondingly results in a high-quality food supplement.

There is also described a food supplement comprising a protein component that is derived from a blood fraction, preferably serum albumin.

The term "protein component" refers in the context of the present invention to the group of free and/or linked amino acids, di-, tri- and polypeptides. Hereby, a distinction is made between so-called "proteinogenic amino acids" and "non-proteinogenic amino acids". Proteinogenic amino acids are amino acids that are incorporated directly into the peptide chain during the translation of the genetic code to a polypeptide. Next to these amino acids, there are also amino acids for which no codon exists, the non-proteinogenic amino acids. They possibly play a role in biological systems and can also occur in de form of peptides.

The protein component of the food supplement comprises one or more free amino acids and di- and/or tripeptides that have been derived from a blood fraction. Di- and tripeptides can be better absorbed and better metabolised by the body of a subject than longer polypeptides and/or more complex proteins, which improves the efficiency of the food supplement. The food supplement hereby shows excellent adaptogenic characteristics with respect to a subject.

Preferably, the amino acids, the di- and tripeptides are degradation products of serum albumin. These degradation products have a high biological value in the human body and are easily absorbed in the intestinal system. Degradation products of serum albumin play an important role in the protein metabolism of the human body. In this way, the food supplement forms a solid supplement to these degradation products and supports the good functioning of the body.

According to a further or other embodiment, the food supplement comprises at least 85 m% of protein components. Thanks to this exceptionally high purity, the food supplement constitutes an extremely good source of amino acids, di- and tripeptides. Preferably, the food supplement comprises at least 90 m%, more preferably at least 95 m%, of protein component.

The "Kjeldahl method" or "Kjeldahl digestion" in the analytic chemistry is a method for the quantitative determination of nitrogen in organic substances, including the nitrogen that is present in the inorganic compounds ammonia and ammonium. The Kjeldahl method is thus an appropriate method for determining the total content of proteins in the food supplement.

The protein component comprises at least 80 m% of di- and/or tripeptides. Since di- and/or tripeptides show an exceptionally good bioavailability and absorption in the human body, a high content of di- and/or tripeptides implies a food supplement with a high bioavailability.

Adaptogenic characteristics comprise characteristics and/or effects from the group of increasing the resistance to fatigue, increasing the resistance to stress, stimulating the immune system, increasing the mental concentration, improving the nitrogen balance, stimulating the production of body proteins, accelerating and/or improving the recovery of tissue damage, inhibiting cell aging, reducing the risk of a depression, supporting the liver function, or combinations thereof. As a result, the food supplement is extremely appropriate for subjects which are exposed to stress factors, subjects wishing to support the immune system, or requiring additional support when sporting, when recovering from injuries, and/or subjects wishing to feel healthier both mentally and/or physically.

Preferably, the food supplement comprises a protein component with at least 90 m% of di- and/or tripeptides. The higher concentration of di- and/or tripeptides further improves the absorption and metabolization of the food supplement by a subject. Most preferably, at least 95 m% of di- and/or tripeptides is present in the protein component.

The food supplement can comprise one or more minerals. Minerals play an essential role in many metabolic processes, for example as a cofactor. The minerals preferably comprise iron. As a result, the food supplement can also be used in cases of iron deficiency. Iron deficiency is usually also at the basis of reduced mental and/or physical performances, therefore supplementing the iron deficiency further supports the adaptogenic function of the food supplement. This iron is preferably heme iron.

The term "heme iron" refers to a specific form of iron, namely bivalent iron, which has a high biological activity. This form of iron is well absorbed by the body and is naturally present in muscle tissue. Trivalent iron, or non-heme iron, that is of a vegetable origin, is absorbed only difficultly by the body. Heme iron is present in the blood in the form of hemine.

Since heme iron is better absorbed by the body, the food supplement shows a high bioavailability as a source of iron. Moreover, heme iron causes less gastro-intestinal discomfort and oxidative stress in a human subject than non-heme iron, which contributes to the adaptogenic characteristics of the food supplement.

The food supplement can also comprise carnitine and/or ornithine. Carnitine, or a di-peptide formed from lysine and methionine, plays a crucial role in the fatty acid metabolism. Carnitine stimulates the fat burning and improves the energy supply to cells. In this way, carnitine contributes to the adaptogenic function of the food supplement. Ornithine is a non-proteinogenic amino acid and is especially active against symptoms of fatigue and increases the vitality in a subject.

The amino acids that are present in the food supplement are chosen from the group of Leucine, Alanine, Phenylalanine, Lysine, Valine, Glutamine, Glutamic acid, Asparagine, Aspartic acid, Histidine, Threonine, Serine, Proline, Tryptophan, Glycine, Thyrosine, Arginine, Methionine, Cysteine, Isoleucine, or combinations thereof.

The food supplement can be a capsule, tablet and/or pellet. This embodiment is easy to take by a subject, and makes the intake of one dose, whether or not on a daily base, very user-friendly. Dosing can thus, depending on the application, be expressed in number of capsules, tablets and/or pellets per intake.

The food supplement is fabricated according to the method of the present invention. The amino acid profile is consequently optimally adapted to the adaptogenic absorption by the human body.

The food supplement can be used in the support and/or improvement of sport performances. The food supplement contributes to an accelerated recovery from injuries, an accelerated build-up of muscle mass, a support of the immune system, an improved focus and/or an improved stress resistance. The food supplement is taken by the subject in a daily dose between 3 and 6 grams for optimally supporting the sport performances.

The food supplement can also be used in cosmetic applications. Cosmetic applications comprise the retardation of the aging process (anti-aging), the improvement of skin and nail quality and/or the support of a healthy body weight. The food supplement is taken by the subject in a daily dose between 7 and 15 grams.

There is also described a feed additive for animals comprising a protein component derived from a blood fraction, preferably serum albumin, which protein component comprises one or more free amino acids, and in which the protein component further comprises di- and/or tripeptides derived from a blood fraction. The feed additive can be added to existing animal feed for improving the energy supply in animals, as well as the quality of the fur and nails. The immune system and the growth of animals is hereby supported. These animals are preferably dogs or horses.

The feed additive is fabricated by means of the method of the present invention.

The method of the invention is further described by example 2. Examples 1, 3 and 4 are for illustrative purposes only.

### EXAMPLES

### Example 1: food supplement with adaptogenic characteristics

The example relates to a food supplement with adaptogenic characteristics with the following composition:

| Component | Concentration (m%) |
|---|---|
| Leucine | 11.22 |
| Alanine | 9.43 |
| Phenylalanine | 8.72 |
| Lysine | 8.32 |
| Valine | 8.10 |
| Glutamine | 6.87 |
| *Glutamic acid* | |
| Asparagine | 6.54 |
| *Aspartic acid* | |
| Histidine | 5.12 |
| Threonine | 5.32 |
| Serine | 5.66 |
| Proline | 4.40 |
| Tryptophan | 4.30 |
| Glycine | 3.89 |
| Thyrosine | 3.65 |
| Arginine | 3.82 |
| Methionine | 1.42 |
| Cysteine | 1.23 |
| Isoleucine | 0.29 |
| Heme iron | 0.03 |
| Carnitine | 0.00* |
| Ornithine | 0.00* |

| | |
|---|---|
| (*) the concentration of carnitine and ornithine in the food supplement is respectively 0.043 mg/100g and 0.039 mg/100g. | |

The food supplement is well absorbed and metabolized by the human body, supports the immune system and counters symptoms of fatigue.

### Example 2: method for the fabrication of a food supplement with adaptogenic characteristics

A food supplement with adaptogenic characteristics is fabricated by means of the following steps:
1. fractioning pork blood until a pure serum albumin is obtained,
2. spray-drying the serum albumin at 180°C, with an average residence time of 30 minutes,
3. dehydrating at 70°C for 8 hours,
4. high-intensity heat treatment at 90°C and 300 kPa, for 0.2 seconds,
5. fractioning to a maximum particle size of 500 µm,
6. physical purification and quality control.

It is advised to go through a complete quality control after each step for obtaining a high-quality supplement. The obtained food supplement has a particularly good amino acid composition, and this in the form of mainly di- and tripeptides.

### Example 3: use of the food supplement at sport performances

The present example illustrates the use of the food supplement from example 2 for supporting and/or improving sport performances.

Ten subjects doing power training, took 6 grams of the food supplement on a daily basis for three months. Compared to a control group, an increase in fat-free mass, or muscle mass, was observed of 2 to 6% per month. Moreover, the subjects were less susceptible to injuries, they were more motivated for doing power training and the muscle mass increased gradually yet more consistent.

At other sports, such as tennis, the subjects reported improved reflexes, a larger feeling of power and an improved motivation at a daily intake of 6 grams of the food supplement.

### Example 4: cosmetic use of the food supplement

The present example illustrates the use of the food supplement from example 2 for improving the skin.

Six subjects with an extremely dry skin took 3 grams of the food supplement on a daily basis for three months. Five of the six subjects reported having a remarkably less dry skin after three months taking the food supplement.

## Claims

1. A method for the fabrication of a food supplement comprising providing a blood fraction of a subject, preferably a mammal, and drying the blood fraction, **characterized in that**, the dried blood fraction is subjected to a high-intensity heat treatment after drying, in which the fraction is heated to a temperature between 80.0 and 110.0°C, at a pressure between 200 and 500 kPa, for 0.1 to 10.0 seconds.

2. The method according to claim 1, whereby the drying of the blood fraction is done by means of spray- and/or freeze-drying.

3. The method according to claim 1 or 2, **characterized in that**, the dried blood fraction undergoes dehydration, which dehydration precedes the high-intensity heat treatment and takes place at a temperature between 60.0 and 80.0°C and at atmospheric pressure, for 5 to 10 hours.

4. The method according to any one of the previous claims 1 to 3, **characterized in that**, the dried blood fraction undergoes a fractionation, which fractionation follows the high-intensity heat treatment and takes place at a maximum particle size of 750 µm.

5. The method according to any one of the previous claims 1 to 4, **characterized in that**, the dried blood fraction undergoes a purification, which purification follows the high-intensity heat treatment and comprises one or more physical purification steps.

6. The method according to any one of the previous claims 2 to 5, **characterized in that**, the spray-drying takes place at a temperature between 170.0 and 190.0 °C.

7. The method according to any one of the previous claims 1 to 6, **characterised in that**, the method comprise the following steps:
a. providing a blood fraction,
b. spray-drying the blood fraction, whereby the temperature during the spray-drying is between 170.0 and 190.0 °C for 25 to 30 minutes,
c. dehydrating the spray-dried fraction at a temperature between 65.0 and 75.0 °C, for 7 to 9 hours, and subsequently
d. high-intensity heat treating the dehydrated fraction, at a temperature between 85.0 and 95.0 °C and a pressure between 290 and 310 kPa, for 0.2 to 0.8 seconds,
e. fractioning the heat-treated fraction to a maximum particle size of 500 µm,
f. physical purification thereof.

## Patentansprüche

1. Verfahren zur Herstellung eines Nahrungsergänzungsmittels, das Bereitstellen einer Blutfraktion eines Subjekts, vorzugsweise eines Säugetiers, und das Trocknen der Blutfraktion umfassend, **dadurch gekennzeichnet, dass** die getrocknete Blutfraktion nach dem Trocknen einer hochintensiven Wärmebehandlung unterzogen wird, wobei die Fraktion bei einem Druck zwischen 200 und 500 kPa 0,1 bis 10,0 Sekunden lang auf eine Temperatur zwischen 80,0 und 110,0 °C erwärmt wird.

2. Verfahren nach Anspruch 1, wobei das Trocknen der Blutfraktion mittels Sprüh- und/oder Gefriertrocknung erfolgt.

3. Verfahren nach Anspruch 1 oder 2, **dadurch gekennzeichnet, dass** die getrocknete Blutfraktion eine Dehydrierung durchläuft, wobei die Dehydrierung der hochintensiven Wärmebehandlung vorangeht und 5 bis 10 Stunden lang bei einer Temperatur zwischen 60,0 und 80,0 °C und einem atmosphärischen Druck stattfindet.

4. Verfahren nach einem der vorhergehenden Ansprüche 1 bis 3, **dadurch gekennzeichnet, dass** die getrocknete Blutfraktion eine Fraktionierung durchläuft, wobei die Fraktionierung der hochintensiven Wärmebehandlung folgt und bei einer maximalen Partikelgröße von 750 µm stattfindet.

5. Verfahren nach einem der vorhergehenden Ansprüche 1 bis 4, **dadurch gekennzeichnet, dass** die getrocknete Blutfraktion eine Reinigung durchläuft, wobei die Reinigung der hochintensiven Wärmebehandlung folgt und einen oder mehrere physikalische Reinigungsschritte umfasst.

6. Verfahren nach einem der vorhergehenden Ansprüche 2 bis 5, **dadurch gekennzeichnet, dass** die Sprühtrocknung bei einer Temperatur zwischen 170,0 und 190,0 °C stattfindet.

7. Verfahren nach einem der vorhergehenden Ansprüche 1 bis 6, **dadurch gekennzeichnet, dass** das Verfahren die folgenden Schritte umfasst:
a. Bereitstellen einer Blutfraktion,
b. Sprühtrocknen der Blutfraktion, wobei die Temperatur während der Sprühtrocknung 25 bis 30 Minuten lang zwischen 170,0 und 190,0 °C liegt,
c. Dehydrieren der sprühgetrockneten Fraktion 7 bis 9 Stunden lang bei einer Temperatur zwischen 65,0 und 75,0 °C und danach
d. hochintensives Wärmebehandeln der dehydrierten Fraktion 0,2 bis 0,8 Sekunden lang bei einer Temperatur zwischen 85,0 und 95,0 °C und bei einem Druck zwischen 290 und 310 kPa,
e. Fraktionieren der wärmebehandelten Fraktion auf eine maximale Partikelgröße von 500 µm,
f. physikalisches Reinigen derselben.

## Revendications

1. Procédé de fabrication d'un complément alimentaire comprenant la fourniture d'une fraction sanguine d'un sujet, de préférence un mammifère, et le séchage de la fraction sanguine, **caractérisé en ce que** la fraction sanguine séchée est soumise à un traitement thermique de haute intensité après le séchage, dans lequel la fraction est chauffée à une température entre 80,0 et 110,0 °C, à une pression entre 200 et 500 kPa, pendant 0,1 à 10,0 secondes.

2. Procédé selon la revendication 1, dans lequel le séchage de la fraction sanguine est effectué au moyen d'un séchage par pulvérisation et/ou d'une lyophilisation.

3. Procédé selon la revendication 1 ou 2, **caractérisé en ce que** la fraction sanguine séchée subit une déshydratation, laquelle déshydratation précède le traitement thermique de haute intensité et a lieu à une température entre 60,0 et 80,0 °C et à pression atmosphérique, pendant 5 à 10 heures.

4. Procédé selon l'une quelconque des revendications précédentes 1 à 3, **caractérisé en ce que** la fraction sanguine séchée subit un fractionnement, lequel fractionnement suit le traitement thermique de haute intensité et a lieu à une taille de particule maximale de 750 µm.

5. Procédé selon l'une quelconque des revendications précédentes 1 à 4, **caractérisé en ce que** la fraction sanguine séchée subit une purification, laquelle purification suit le traitement thermique de haute intensité et comprend une ou plusieurs étapes de purification physique.

6. Procédé selon l'une quelconque des revendications précédentes 2 à 5, **caractérisé en ce que** le séchage par pulvérisation a lieu à une température entre 170,0 et 190,0 °C.

7. Procédé selon l'une quelconque des revendications précédentes 1 à 6, **caractérisé en ce que** le procédé comprend les étapes suivantes :
a. fourniture d'une fraction sanguine,
b. séchage par pulvérisation de la fraction sanguine, dans lequel la température durant le séchage par pulvérisation est entre 170,0 et 190,0 °C pendant 25 à 30 minutes,
c. déshydratation de la fraction séchée par pulvérisation à une température entre 65,0 et 75,0 °C, pendant 7 à 9 heures, et ensuite
d. traitement thermique de haute intensité de la fraction déshydratée, à une température entre 85,0 et 95,0 °C et une pression entre 290 et 310 kPa, pendant 0,2 à 0,8 seconde,
e. fractionnement de la fraction traitée thermiquement à une taille de particule maximale de 500 µm,
f. purification physique de celle-ci.
